(19) Europäisches Patentamt
European Patent Office
Office européen des brevets

(11) **EP 4 563 118 A1**

(12) **EUROPÄISCHE PATENTANMELDUNG**

(43) Veröffentlichungstag:
**04.06.2025 Patentblatt 2025/23**

(21) Anmeldenummer: **23213107.8**

(22) Anmeldetag: **29.11.2023**

(51) Internationale Patentklassifikation (IPC):
*A61C 13/00* (2006.01)   *A61C 13/08* (2006.01)
*A61C 13/083* (2006.01)   *A61C 13/09* (2006.01)
*A61K 6/818* (2020.01)   *B33Y 10/00* (2015.01)
*B33Y 40/20* (2020.01)   *B33Y 80/00* (2015.01)

(52) Gemeinsame Patentklassifikation (CPC):
**A61C 13/082; A61C 13/0019; A61C 13/083;
A61C 13/09; A61K 6/16; B33Y 10/00; B33Y 40/20;
B33Y 80/00;** A61K 6/802; B33Y 70/00

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(71) Anmelder: **Ivoclar Vivadent AG**
**9494 Schaan (LI)**

(72) Erfinder:
• **John, Hendrik**
**9470 Buchs (CH)**
• **Rothbrust, Frank**
**6822 Röns (AT)**
• **Krolikowski, Sebastian**
**8805 Richterswil (CH)**
• **Ritzberger, Christian**
**9472 Grabs (CH)**

(74) Vertreter: **Baldus, Oliver**
**Splanemann**
**Rumfordstrasse 7**
**80469 München (DE)**

(54) **VOREINGEFÄRBTER SCHLICKER FÜR KERAMISCHEN MULTI-COLOR-INKJET-3D-DRUCK**

(57)     Die vorliegende Erfindung betrifft ein Verfahren zum Herstellen einer dentalen Restauration durch Strahldruck, mit den Schritten eines Strahldruckens (S101) eines Dentinkerns der dentalen Restauration mittels einer Mehrzahl opaker keramischer Schlicker, die zueinander unterschiedliche Farben aufweisen; und eines Strahldruckens (S102) des Zahnschmelzes der dentalen Restauration mittels eines transluzenten keramischen Schlickers.

**Fig. 4**

**Beschreibung**

Die vorliegende Erfindung betrifft ein Verfahren zum Herstellen einer dentalen Restauration durch Strahldruck und ein Herstellungsgerät zum Herstellen einer dentalen Restauration durch Strahldruck.

Heutzutage werden bereits verschieden eingefärbte, lösungsmittelbasierte Keramikschlicker, beispielsweise aus Zirkondioxid, selektiv mittels eines Strahlverfahrens (Inkjet-Verfahren) tröpfchenweise in einer Vielzahl von Schichten aufeinander aufgetragen und getrocknet. Die Verarbeitung von hochgefülltem, keramischem Schlicker stellt jedoch hohe Anforderungen an den eingesetzten Druckkopf in Bezug auf eine Partikelgröße, einen Füllgrad, eine Viskosität, Abrasion und Korrosion. Das Strahldrucken (Jetten) von wässrigen Keramikschlickern stellt eine Alternative zu den lösungsmittelbasierten Schlickern dar.

Es ist die technische Aufgabe der vorliegenden Erfindung, keramische, dentale Restaurationen mittels eines dreidimensionalen Druckverfahrens farbgetreu und natürlich aussehend zu erzeugen.

Diese Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren zum Herstellen einer dentalen Restauration durch Strahldruck gelöst, mit den Schritten eines Strahldruckens eines Dentinkerns der dentalen Restauration mittels einer Mehrzahl opaker keramischer Schlicker, die zueinander unterschiedliche Farben aufweisen; und eines Strahldruckens des Zahnschmelzes der dentalen Restauration mittels eines transluzenten keramischen Schlickers. Als keramische Schlicker können wässrige oder lösungsmittelbasierte Schlicker verwendet werden. Für das Verfahren wird eine begrenzte Anzahl voreingefärbter Schlicker bereitgestellt, um die Anzahl der benötigten Druckköpfe zu verringern und dennoch ein hoch ästhetisches und funktionelles Ergebnis für die dentale Restauration zu erzielen.

Ein opaker Schlicker ist ein Schlicker, der nach dem Sinterprozess kein Licht durchlässt und undurchsichtig ist. Demgegenüber ist ein transluzenter Schlicker ein Schlicker, der nach dem Sinterprozess Licht teilweise durchlässt, aber keine klaren Bilder oder Formen erkennen lässt. Die reziproke (wechselseitige) Eigenschaft zur Transluzenz ist die Opazität (Lichtundurchlässigkeit). Weist ein gesinterter Schlicker, also die finale Keramik, eine hohe Transluzenz auf, so hat dieser in der dentalen Restauration eine geringe Opazität und umgekehrt. Vor dem Sinterprozess sind die optischen Eigenschaften der jeweiligen Schlicker im Allgemeinen andere. Die Transluzenz oder Opazität entsteht erst durch die chemische Zusammensetzung des Schlickers in Verbindung mit dem Sinterprozess in der final gesinterten Keramik. Die optischen Eigenschaften der Transluzenz oder der Opazität sind also das Ergebnis des Sinterprozesses.

Ein opaker Schlicker umfasst beispielsweise Yttriumstabilisierte $ZrO_2$-Partikel mit einem $Y_2O_3$-Anteil von 1.5 bis 4.5 Mol.%, bevorzugt von 2 bis 4 Mol.% und besonders bevorzugt 2.5 bis 3.5 Mol.%.

Ein transluzenter Schlicker umfasst beispielsweise Yttriumstabilisierte $ZrO_2$-Partikel mit einem $Y_2O_3$-Anteil von 4.0 bis 8.0 Mol.%, bevorzugt 4.0 bis 7.0 Mol.% und besonders bevorzugt 4.0 bis 6.0 Mol.%.

In einer technisch vorteilhaften Ausführungsform des Verfahrens ist ein Yttrium-Anteil des transluzenten keramischen Schlickers höher als der Yttrium-Anteil eines der opaken keramischen Schlicker. Dadurch wird beispielsweise der technische Vorteil erreicht, dass besonders geeignete Schlicker verwendet werden, um in der gesinterten dentalen Restauration Bereiche mit unterschiedlicher Transluzenz, hoch im Zahnschmelz und niedrig im Dentinkern, zu erzeugen.

In einer weiteren technisch vorteilhaften Ausführungsform des Verfahrens wird der Dentinkern der dentalen Restauration zusätzlich mittels eines eingefärbten transluzenten Schlickers strahlgedruckt. Dadurch wird beispielsweise der technische Vorteil erreicht, dass das natürliche Erscheinungsbild der dentalen Restauration noch weiter verbessert wird.

In einer weiteren technisch vorteilhaften Ausführungsform des Verfahrens bilden die unterschiedlichen Farben der Schlicker für den Dentinkern ein Farbschema mit einem eingeschränkten Farbraum. Der eingeschränkte Farbraum ist für die Farbgebung der dentalen Restauration angepasst. Würde ein Farbschema zur Abdeckung des gesamten Farbraums verwendet, wäre eine höhere Anzahl an Druckköpfen erforderlich. Auch die Herstellung dieser verschiedenen Schlicker ist aufwendig. Dadurch wird beispielsweise der technische Vorteil erreicht, dass die Anzahl der Druckköpfe verringert und die Anzahl der vorgehaltenen Schlicker reduziert werden kann.

In einer weiteren technisch vorteilhaften Ausführungsform des Verfahrens wird ein Bereich der dentalen Restauration mit einem Farbzwischenwert und/oder Transluzenzzwischenwert durch Mischen von mindestens zwei Schlickern erzeugt. Dadurch wird beispielsweise der technische Vorteil erreicht, dass die dentale Restauration mit Farbwerten oder Transluzenzwerten erzeugt werden kann, die nicht denen der eingesetzten Schlicker entsprechen.

In einer weiteren technisch vorteilhaften Ausführungsform des Verfahrens wird das Mischen durch das Erzeugen eines zweidimensionales Dithering-Musters für eine Schicht der dentalen Restauration umgesetzt. Das Dithering-Muster gibt an, wie die unterschiedlichen Schlicker in einer zweidimensionalen Ebene angeordnet sind, um den Farbzwischenwert und/oder Transluzenzzwischenwert zu erzeugen. Dadurch wird beispielsweise ebenfalls der technische Vorteil erreicht, dass die dentale Restauration mit einem natürlichen Erscheinungsbild hergestellt werden kann.

In einer weiteren technisch vorteilhaften Ausführungsform des Verfahrens werden in aufeinanderfolgenden Schichten der dentalen Restauration unterschiedliche zweidimensionale Dithering-Muster verwendet. Dadurch wird beispielsweise der technische Vorteil erreicht, dass das Entstehen von Streifen- oder Moiré-Mustern in der dentalen Restauration verhindert wird.

In einer weiteren technisch vorteilhaften Ausführungsform des Verfahrens ist jedem Aufnahmebehälter für Schlicker ein Druckkopf zugeordnet. Dadurch wird beispielsweise der technische Vorteil erreicht, dass die dentale Restauration auf einfache und schnelle Weise gedruckt werden kann.

In einer weiteren technisch vorteilhaften Ausführungsform des Verfahrens wird die dentale Restauration in einem Sinterofen gesintert. Dadurch wird beispielsweise der technische Vorteil erreicht, dass eine dentale Restauration mit hoher Festigkeit hergestellt werden kann.

In einer weiteren technisch vorteilhaften Ausführungsform des Verfahrens wird die hergestellte dentale Restauration vor einem Sinterprozess einem Trocknungs- und/oder Entbinderungsschritt unterzogen. Dieser Trocknungs- und/oder Entbinderungsschritt kann in einem separaten thermischen Prozess erfolgen oder ist ein im Sinterprozess vorgelagerter Prozessschritt. Üblicherweise wird das Trocknen bei Temperaturen von 25°C bis 200°C, bevorzugt 30°C bis 180°C und besonders bevorzugt 40°C bis 150°C getrocknet. Dabei kann zusätzlich die Luftfeuchtigkeit zwischen 10 bis 90%, bevorzugt 15 bis 85% und besonders bevorzugt 20 bis 80% reguliert werden. Üblicherweise wird bei Temperaturen von 50°C bis 600°C, bevorzugt zwischen 100°C bis 600°C und besonders bevorzugt bei 200°C-600°C entbindert. Die Aufheizgeschwindigkeiten liegen zwischen 0.1 bis 10 K/min, bevorzugt zwischen 0.2 bis 10K/min und besonders bevorzugt zwischen 0.5 bis 10 K/min.

Gemäß einem zweiten Aspekt wird die technische Aufgabe durch ein Herstellungsgerät zum Herstellen einer dentalen Restauration durch Strahldruck gelöst; mit einer Mehrzahl von Aufnahmebehältern zum Aufnehmen opaker keramischer Schlicker für einen Dentinkern, die zueinander unterschiedliche Farben aufweisen; und mindestens einem Aufnahmebehälter zum Aufnehmen eines transluzenten keramischen Schlickers für den Zahnschmelz. Das Herstellungsgerät verwendet ein dreidimensionales Multi-Color-Druckverfahren mittels Material-Jetting keramischer Schlicker. Durch das Herstellungsgerät werden die gleichen technischen Vorteile wie durch das Verfahren nach dem ersten Aspekt erreicht.

In einer technisch vorteilhaften Ausführungsform des Herstellungsgeräts umfasst das Herstellungsgerät jeweils einen Druckkopf je Aufnahmebehälter. Dadurch wird beispielsweise der technische Vorteil erreicht, dass die dentale Restauration auf einfache und schnelle Weise gedruckt werden kann.

In einer weiteren technisch vorteilhaften Ausführungsform des Herstellungsgeräts umfasst das Herstellungsgerät mindestens zwei Aufnahmebehälter für Schlicker, wie beispielsweise fünf, sechs, sieben oder acht Aufnahmebehälter für Schlicker. Dadurch wird beispielsweise der technische Vorteil erreicht, dass eine geringe Anzahl von Druckköpfen und Aufnahmebehältern zur Herstellung naturgetreuer dentaler Restaurationen verwendet werden kann.

In einer weiteren technisch vorteilhaften Ausführungsform des Herstellungsgeräts bilden die unterschiedlichen Farben der Schlicker für den Dentinkern ein Farbschema mit einem eingeschränkten Farbraum. Dadurch wird beispielsweise der technische Vorteil erreicht, dass eine geringere Anzahl von Druckköpfen oder Aufnahmebehältern als bei einem vollständigen Farbschema verwendet werden kann.

In einer weiteren technisch vorteilhaften Ausführungsform des Herstellungsgeräts umfasst das Herstellungsgerät ein Dithering-Modul zum Berechnen von Farbzwischenwerten oder Transluzenzzwischenwerten durch Mischen von mindestens zwei Schlickern. Dadurch wird beispielsweise der technische Vorteil erreicht, dass die dentale Restauration mit Farbwerten oder Transluzenzwerten erzeugt werden kann, die nicht denen der eingesetzten Schlicker entsprechen.

In einer weiteren technisch vorteilhaften Ausführungsform des Herstellungsgeräts ist das Dithering-Modul ausgebildet, in aufeinanderfolgenden Schichten der dentalen Restauration unterschiedliche zweidimensionale Dithering-Muster zu verwenden. Dadurch wird beispielsweise der technische Vorteil erreicht, dass das Entstehen von Streifen- oder Moiré-Mustern in der dentalen Restauration verhindert wird.

Ausführungsbeispiele der Erfindung sind in den Zeichnungen dargestellt und werden im Folgenden näher beschrieben.

Es zeigen:

Fig. 1    eine Darstellung unterschiedlicher Bereiche eines Schneidezahns;

Fig. 2    eine schematische Darstellung einer dentalen Restauration;

Fig. 3    eine schematische Darstellung eines Herstellungsgeräts zum Herstellen einer dentalen Restauration durch Strahldruck; und

Fig. 4    ein Blockdiagramm eines Verfahrens zum Herstellen der dentalen Restauration durch Strahldruck.

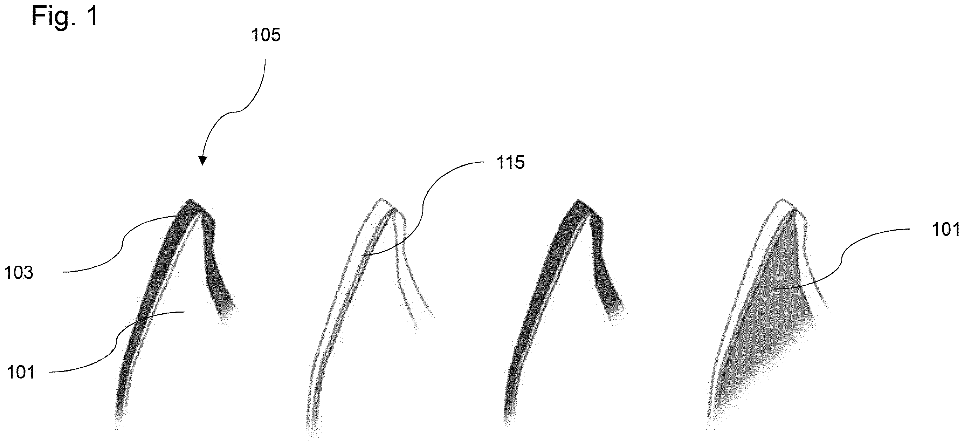

Fig. 1 zeigt eine Darstellung unterschiedlicher Bereiche eines Zahns 105. Der Zahn 105 umfasst einen inneren Dentinkern 101 und einen äußeren Zahnschmelz 103. Zwischen dem Dentinkern 101 und dem Zahnschmelz 103 befindet sich ein Zwischenbereich 115.

Für die Grundeinfärbung des Zahns 105 ist der opake, d.h. undurchsichtige Dentinkern 101 verantwortlich. Dieser scheint durch den Zahnschmelz 103 der Schneide hindurch. Der Zahnschmelz 103 ist durchscheinend, d.h. transluzent. Transluzenz ist die partielle Lichtdurchlässigkeit eines Körpers. Um eine dentale Restauration möglichst naturgetreu aussehen zu lassen, wird dieser Aufbau des Zahns 105 auch in der künstlichen dentalen Restauration verwendet. Hierzu werden Materialien mit unterschiedlichen optischen Eigenschaften bei der Herstellung der dentalen Restauration verwendet.

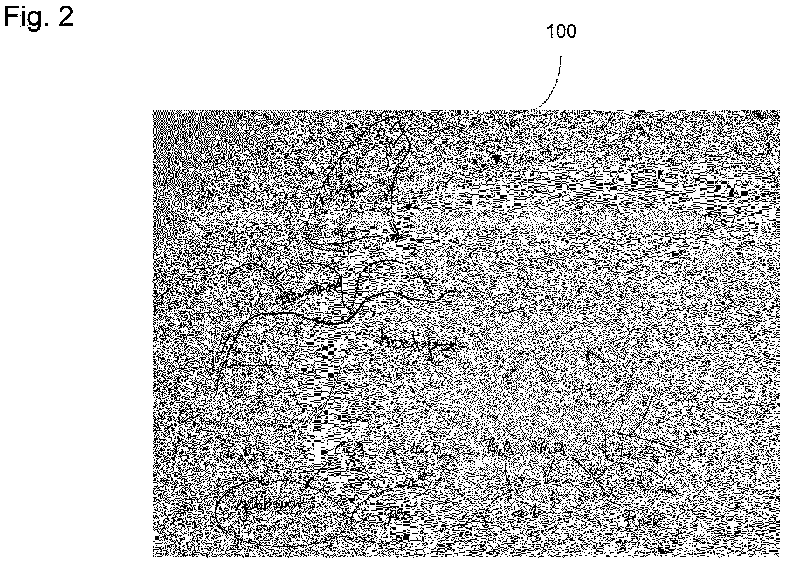

Fig. 2 zeigt eine schematische Darstellung einer dentalen Restauration 100. Die dentale Restauration 100 dient als Zahnersatz und ist beispielsweise durch eine Brücke, Krone, Veneer, Inlay, Onlay, Abutment, Voll- oder Teilprothese gebildet. Die dentale Restauration 100 wird beispielsweise mittels unterschiedlich eingefärbter, keramischer Schlicker aufgebaut, die in geringen Mengen färbende Komponenten enthalten können, wie z.B. $Fe_2O_3$, $Cr_2O_3$, $Mn_2O_3$, $Tb_2O_3$, $Pr_2O_3$, $Er_2O_3$, $Co_3O_4$, $NiO$, $TiO_2$, $CeO_2$. Hierzu werden diese Schlicker bei der räumlichen Herstellung der dentalen Restauration 100 mittels eines Strahldruckverfahrens selektiv in den jeweiligen Bereichen eingesetzt.

Die dentale Restauration 100 wird dabei durch aufeinander folgende Schichten aufgebaut, die aufeinander gedruckt werden. Dabei können die Keramikpulver der Schlicker bereits in vorgegebenen Farben und Transluzenzen bereitgestellt sein. Durch die Mischung dieser Schlicker ergeben sich die Soll-Zahnfarbe und die Soll-Transluzenz der dentalen Restauration 100 in dem jeweiligen räumlichen Bereich.

Nach dem selektiven Materialauftrag einer Schicht des Schlickers mittels des Strahldruckverfahrens wird diese Schicht rissfrei getrocknet, indem das Wasser oder das Lösungsmittel verdampft wird. Zurück bleibt eine poröse Weissling-Schicht, die eine Schichtdicke von 1 um bis 50 um und eine Dichte von mindestens 2.5 g/cm$^3$ aufweist. Dieser Prozess wird wiederholt, bis die gesamte dentale Restauration 100 schichtweise räumlich aufgebaut ist.

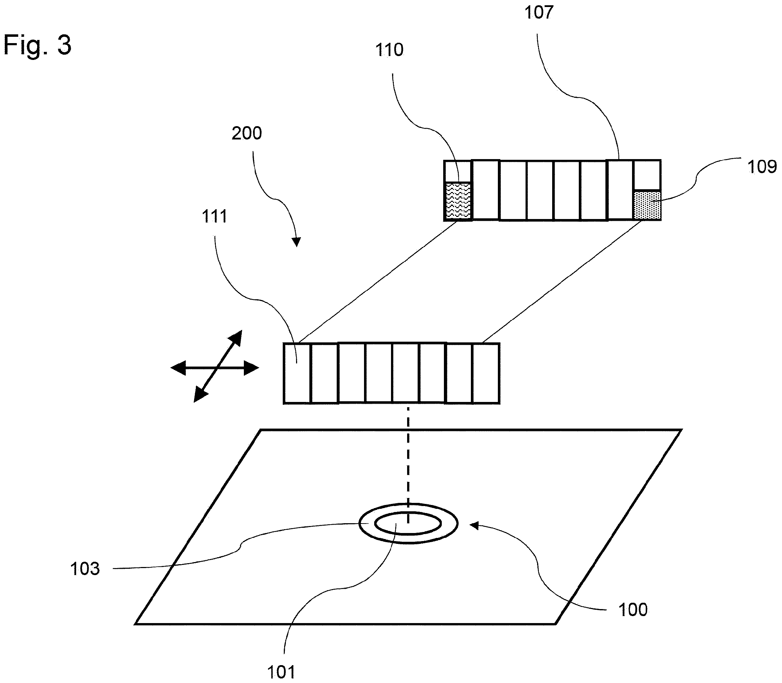

Fig. 3 zeigt eine schematische Darstellung eines Herstellungsgeräts 200 zum Herstellen der dentalen Restauration 100 durch Strahldruck (Inkjet-Druck) von wässrigen oder lösungsmittelbasierten Schlickern 109. Um keramische, dentale Multimaterial- und Multicolor-Restaurationen 100 mittels Strahldruck additiv zu fertigen, werden voreingefärbte Schlicker 109 verarbeitet.

Das Herstellungsgerät 200 umfasst eine Mehrzahl von Aufnahmebehältern 107, in denen jeweils ein keramischer Schlicker 109 mit unterschiedlichen optischen Eigenschaften für den Dentinkern 101 angeordnet ist. Zudem umfasst das Herstellungsgerät 200 zumindest einen Aufnahmebehälter 107, in dem ein transluzenter keramischer Schlicker 109 zum Herstellen des Zahnschmelzes 103 aufgenommen ist.

Die keramischen Schlicker 109 werden jeweils mittels eines zugeordneten Druckkopfes 111 tröpfchenweise in mehreren Schichten aufgebracht, um die dentale Restauration 100 schichtweise räumlich aufzubauen. Der Druckkopf 111 ist in zwei Richtungen beweglich, so dass der Schlicker 109 an jeder Position aufgedruckt werden kann. Für den selektiven Materialauftrag werden Schlicker 109 mit einem Tropfenvolumen von typischerweise 10 bis 100 pL verwendet, durch deren Einsatz der zeitlich aufwändige Entbinderungsprozess entfällt.

Zum Ausstoßen der Tropfen werden elektrisch gesteuerte Piezoelemente verwendet. Alternativ kann auch das Bubblejet-Verfahren verwendet werden.

Das Herstellungsgerät 200 stellt eine verringerte Anzahl an voreingefärbten und Yttrium-dotierten Schlickern 109 bereit, um die Anzahl der Druckköpfe 111 zu minimieren und dennoch ein ästhetisches und funktionelles Ergebnis der dentalen Restauration 100 zu erzielen.

Die gewünschte Zahnfarbe wird aus den bereits voreingefärbten Schlickern 109 zusammengesetzt und gemischt. Dabei wird ein subtraktives Farbsystem verwendet, das einen eingeschränkten, dentalen Farbraum (Dental Color Gamut) aufspannt. Die Farbmischung, das dreidimensionale Halftoning oder Dithering dieser voreingefärbten Schlicker 109 in verschiedenen Verhältnissen entsteht dann in dem speziellen, dentalen Farbraum (Gamut), der die gängigen Zahnfarben, jedoch nicht alle Farben abdeckt.

Dabei werden unterschiedlich eingefärbte Schlicker 109 über einen 3D-Dithering-Algorithmus in einer Schicht selektiv aufgetragen, der durch ein Dithering-Modul 113 ausgeführt wird. Das Dithering-Modul 113 umfasst hierzu einen Prozessor zum Ausführen des 3D-Dithering-Algorithmus und einen digitalen Datenspeicher zum Speichern der berechneten Mischungsverhältnisse. Der Prozessor umfasst jedes Hardwaresystem, jede Komponente oder jeden Mechanismus, der Daten, Signale oder andere Information verarbeitet. Ein Prozessor kann ein System mit einer zentralen Datenverarbeitungseinheit (CPU), mehreren Datenverarbeitungseinheiten (MPU), eine dedizierte elektrische Schaltung zum Erreichen der Funktionalität oder andere Systeme umfassen. Der Datenspeicher kann Festplatten, Flash-Speicherkarten, wahlfreie Zugriffsspeicher (RAM), Nur-Lesespeicher (ROM) umfassen.

Bei dem Dithering werden die Schlicker 109 unterschiedlicher Farben in einem bestimmten Verhältnis und zweidimensionalen Druckmuster in der Druckebene selektiv mittels des Strahldruckverfahrens aufgetragen. Der 3D-Dithering-Algorithmus berechnet zudem, dass nicht gleiche zweidimensionale Dithering-Muster in mehreren Schichten übereinander aufgetragen werden. Dadurch können bei senkrechten Flächen optische Artefakte wie Streifen- oder Moiré-Muster verhindert werden. Die Farbmischung, das dreidimensionale Halftoning oder Dithering dieser voreingefärbten Schlicker 109 in verschiedenen Verhältnissen entsteht innerhalb des speziellen, dentalen Farbraums (Gamut), der die gängigen Zahnfarben, jedoch nicht alle allgemeinen Farben abdeckt.

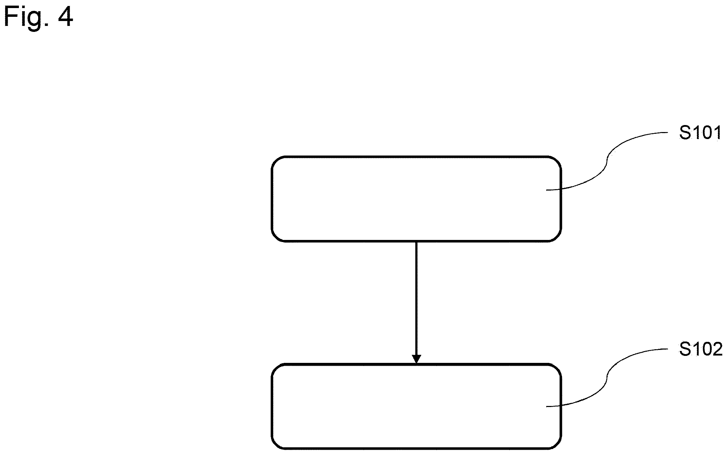

Fig. 4 zeigt ein Blockdiagramm eines Verfahrens zum Herstellen der dentalen Restauration 100 durch Strahldruck. In Schritt S101 wird der Dentinkern 101 der dentalen Restauration 100 mittels einer Mehrzahl opaker keramischer Schlicker 109 strahlgedruckt, die zueinander unterschiedliche Farben aufweisen. In Schritt S102 wird der Zahnschmelz 103 der dentalen Restauration 100 mittels eines transluzenten keramischen Schlickers 109 strahlgedruckt. Danach wird die so aufgebaute dentale Restauration 100 in einem Sinterofen gesintert.

Es gibt Schlicker 109, die für den Aufbau des Zahnschmelzes 103 der Schneide verwendet werden, und Schlicker 109, die für den Aufbau des Dentinkerns 101 verwendet werden. Der Unterschied dieser Schlicker 109 liegt in der Transluzenz des Materials, die für den Zahnschmelz 103 um ein Vielfaches höher ist als für den Dentinkern 101.

Beispielsweise können Schlicker 109 in sieben verschiedenen Grundeinfärbungen verwendet werden, von denen zwei für den Zahnschmelz 103 vorgesehen sind und ein zusätzliches Stützmaterial 110 für Unterstützungsstrukturen (Support). Bei dem Stützmaterial handelt es sich um organische Komponenten handelt, wie beispielsweise um Wachse, Paraffine oder Russ-Schlicker.

1   Weiß, hoch-transluzent (Schneide/Inzisalbereich), höherer Y-Dotierungsanteil
2   Gelbbraun, hoch-transluzent (Schneide/Inzisalbereich), höherer Y-Dotierungsanteil
3   Weiß, opak, hoch-fest (Dentinbereich)
4   Rosa, transluzent, fest (Dentinbereich, auch zur Detaillierung für die Schneide geeignet)
5   Grau, transluzent, fest (Dentinbereich auch zur Detaillierung für Schneide geeignet)
6   Gelb, transluzent, fest (Dentinbereich)
7   Gelbbraun, opak, hoch-fest (Dentinbereich)
8   Stützmaterial

In einer weiteren Ausführungsform gibt es nur einen einzigen transluzenten Schlicker 109 und fünf verschiedene Schlicker 109, die für die Farbgebung im Dentinkern 101 bereitgestellt werden. Dies hat den technischen Vorteil, dass bereits sieben Druckköpfe 111 (inkl. Stützmaterial 110) ausreichend sind.

1   Schneide hoch-transluzent (8-9 Y) (Schneide/Inzisalbereich)
2   Weiß, opak, hoch-fest (Dentinbereich)
3   Rosa, transluzent, fest (Dentinbereich, auch zur Detaillierung für Schneide geeignet)
4   Grau, transluzent, fest (Dentinbereich, auch zur Detaillierung für Schneide geeignet)
5   Gelb, transluzent, fest (Dentinbereich, auch zur Detaillierung für Schneide geeignet)
6   Gelbbraun, opak, hoch-fest (Dentinbereich)
7   Stützmaterial

Für die Grundeinfärbung ist der Dentinkern 101 verantwortlich und scheint durch den Zahnschmelz 103 der Schneide hindurch. Im Extremfall ist die Schneide farblos und hoch-transluzent. Hierfür werden zur Farbgebung und für den zahnspezifischen Kern-/Schalenaufbau (Dentin/Schneide) speziell voreingefärbte und Yttrium-dotierte Schlicker 109 vorgehalten.

Im Falle von ZrO2-Schlickern 109 können für die verschiedenen Festigkeiten unterschiedliche Yttrium-dotierte Keramikpulver (3 mol% Yttrium - 3Y-TZP, 4 mol% Yttrium - 4Y-TZP, 5 mol% Yttrium - 5Y-TZP) zum Einsatz kommen. Dabei ist die Yttrium-Dotierung auch für den Grad der Transluzenz ursächlich. Die unterschiedlichen Yttrium-dotierten Keramikpulver weisen unterschiedliche Eigenschaften auf.

```
3Y-TZP = niedrige Transluzenz / hohe Festigkeit

4Y-TZP = mittlere Transluzenz / mittlere Festigkeit

5Y-TZP = hohe Transluzenz / niedrige Festigkeit
```

Würden jedoch unterschiedlich voreingefärbte Schlicker 109 verwendet, die alle auch eine unterschiedliche Transluzenz und/oder mechanische Eigenschaften aufweisen, würde für jeden dieser Schlicker 109 ein eigener Druckkopf 111 verwendet. Wird beispielsweise ein allgemeines Vierfarb-Farbschema eingesetzt, um den gesamten Farbraum abzudecken und zudem Schlicker mit unterschiedlichen Transluzenzen oder Festigkeiten in drei Varianten eingesetzt, so sind 3 x 4 = 12 Schlicker 109 in 12 Druckköpfen erforderlich. Auch die Herstellung dieser verschiedenen Schlicker 109 ist aufwendig.

Die voreingefärbten Schlicker 109 mit ihren unterschiedlichen Yttrium-Anteilen sollten auf ein einheitliches Sinterverhalten abgestimmt sein. Die Einstellung des Sinterverhaltens der einzelnen Schichten kann durch gezielte Zugabe von Sinteraktivatoren oder Sinterinhibitoren erfolgen. Sinteraktivatoren sind z.B. $Zn2+$- oder $Mg2+$-Ionen die in Form von löslichen Salzen, wie z.B. $Zn(NO3)2*6H2O$ oder $Mg(NO3)*H2O$ der Färbelösung zugegeben werden können. Sinterinhibitoren sind z.B. $Al3+$ oder $Y3+$, die in Form von löslichen Salzen, wie z.B. $Al(NO3)3 * 9H2O$ oder $Y(NO3)3 * 6H2O$ der Färbelösung zugegeben werden können.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

BEZUGSZEICHENLISTE

| | |
|---|---|
| 100 | Dentale Restauration |
| 101 | Dentinkern |
| 103 | Zahnschmelz |
| 105 | Zahn |
| 107 | Aufnahmebehälter |
| 109 | Schlicker |
| 110 | Stützmaterial |
| 111 | Druckkopf |
| 113 | Dithering-Modul |
| 115 | Zwischenbereich |

**Patentansprüche**

1. Verfahren zum Herstellen einer dentalen Restauration (100) durch Strahldruck, mit den Schritten:

   - Strahldrucken (S101) eines Dentinkerns (101) der dentalen Restauration (100) mittels einer Mehrzahl opaker keramischer Schlicker (109), die zueinander unterschiedliche Farben aufweisen; und
   - Strahldrucken (S102) des Zahnschmelzes (103) der dentalen Restauration (100) mittels eines transluzenten keramischen Schlickers (109).

2. Verfahren nach Anspruch 1, wobei ein Yttrium-Anteil des transluzenten keramischen Schlickers (109) höher als der Yttrium-Anteil eines der opaken keramischen Schlicker (109) ist.

3. Verfahren nach einem der vorangehenden Ansprüche, wobei der Dentinkern (101) der dentalen Restauration (100) zusätzlich mittels eines eingefärbten transluzenten Schlickers (109) strahlgedruckt wird.

4. Verfahren nach einem der vorangehenden Ansprüche, wobei die unterschiedlichen Farben der Schlicker (109) für den Dentinkern (101) ein Farbschema mit einem eingeschränkten Farbraum bilden.

5. Verfahren nach einem der vorangehenden Ansprüche, wobei ein Bereich der dentalen Restauration (100) mit einem Farbzwischenwert und/oder Transluzenzzwischenwert durch Mischen von mindestens zwei Schlickern (109) erzeugt wird.

6. Verfahren nach Anspruch 5, wobei das Mischen durch das Erzeugen eines zweidimensionales Dithering-Musters für eine Schicht der dentalen Restauration (100) umgesetzt wird.

7. Verfahren nach Anspruch 6, wobei in aufeinanderfolgenden Schichten der dentalen Restauration unterschiedliche zweidimensionale Dithering-Muster verwendet werden.

8. Verfahren nach einem der vorangehenden Ansprüche, wobei jedem Aufnahmebehälter (107) für Schlicker (109) ein Druckkopf (111) zugeordnet ist.

9. Verfahren nach einem der vorhergehenden Ansprüche, wobei die hergestellte dentale Restauration (100) vor einem Sinterprozess einem Trocknungs- und/oder Entbinderungsschritt unterzogen wird.

10. Verfahren nach einem der vorangehenden Ansprüche, wobei die dentale Restauration (100) in einem Sinterofen gesintert wird.

11. Herstellungsgerät (200) zum Herstellen einer dentalen Restauration (100) durch Strahldruck; mit:

   - einer Mehrzahl von Aufnahmebehältern (107) zum Aufnehmen opaker keramischer Schlicker (109) für einen Dentinkern (101), die zueinander unterschiedliche Farben aufweisen; und
   - mindestens einem Aufnahmebehälter (107) zum Aufnehmen eines transluzenten keramischen Schlickers (109) für den Zahnschmelz (103).

12. Herstellungsgerät (200) nach Anspruch 11, wobei das Herstellungsgerät (200) jeweils einen Druckkopf (111) je Aufnahmebehälter (107) umfasst.

13. Herstellungsgerät (200) nach einem der Ansprüche 10 bis 12, wobei die unterschiedlichen Farben der Schlicker (109) für den Dentinkern (101) ein Farbschema mit einem eingeschränkten Farbraum bilden.

14. Herstellungsgerät (200) nach einem der Ansprüche 10 bis 13, wobei das Herstellungsgerät (200) ein Dithering-Modul (113) zum Berechnen von Farbzwischenwerten und/oder Transluzenzzwischenwerten durch Mischen von mindestens zwei Schlickern (109) umfasst.

15. Herstellungsgerät (200) nach Anspruch 14, wobei das Dithering-Modul (113) ausgebildet ist, in aufeinanderfolgenden Schichten der dentalen Restauration (100) unterschiedliche zweidimensionale Dithering-Muster zu verwenden.

Fig. 1

Fig. 2

100

Fig. 3

Fig. 4

S101

S102

Europäisches
Patentamt

European
Patent Office

Office européen
des brevets

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 529 694 A1 (IVOCLAR VIVADENT AG [LI]) 5. Dezember 2012 (2012-12-05) | 1,3-5, 8-13 | INV. A61C13/00 |
| Y | * Absätze [0023] - [0025], [0028], | 2 | A61C13/08 |
| A | [0032], [0034], [0041], [0042], [0092] * | 6,7,14, 15 | A61C13/083 A61C13/09 A61K6/818 |
| X | EP 3 659 547 A1 (IVOCLAR VIVADENT AG [LI]) 3. Juni 2020 (2020-06-03) | 11 | B33Y10/00 B33Y40/20 |
| Y | * Absätze [0014], [0015], [0020], | 2 | B33Y80/00 |
| A | [0027], [0056], [0066] * | 6,7,14, 15 | |

RECHERCHIERTE
SACHGEBIETE (IPC)

A61C
A61K
B33Y

Der vorliegende Recherchenbericht wurde für alle Patentansprüche erstellt

| Recherchenort | Abschlußdatum der Recherche | Prüfer |
|---|---|---|
| **München** | **10. April 2024** | **Kerner, Bodo** |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 23 21 3107

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-04-2024

| Im Recherchenbericht angeführtes Patentdokument | Datum der Veröffentlichung | Mitglied(er) der Patentfamilie | | Datum der Veröffentlichung |
|---|---|---|---|---|
| EP 2529694 A1 | 05-12-2012 | EP | 2529694 A1 | 05-12-2012 |
| | | JP | 2012250022 A | 20-12-2012 |
| | | US | 2012308837 A1 | 06-12-2012 |
| EP 3659547 A1 | 03-06-2020 | CN | 111233466 A | 05-06-2020 |
| | | EP | 3659547 A1 | 03-06-2020 |
| | | ES | 2906273 T3 | 18-04-2022 |
| | | JP | 7128168 B2 | 30-08-2022 |
| | | JP | 2020083755 A | 04-06-2020 |
| | | KR | 20200066225 A | 09-06-2020 |
| | | KR | 20230040967 A | 23-03-2023 |
| | | PL | 3659547 T3 | 21-03-2022 |
| | | US | 2020171699 A1 | 04-06-2020 |
| | | US | 2023347548 A1 | 02-11-2023 |

EPO FORM P0461

Für nähere Einzelheiten zu diesem Anhang : siehe Amtsblatt des Europäischen Patentamts, Nr.12/82